# EUROPEAN PATENT APPLICATION

(11) **EP 0 972 832 A1**
(43) Date of publication of application: **19.01.2000**
(21) Application number: 98113257.4
(22) Date of filing: 16.07.1998
(51) Int. Cl.: C12N 15/12, C12P 21/02, C12Q 1/68, C07K 14/435

(54) **New gene with reduced expression in metastatic human melanoma cells and the protein coded thereby, methods of production and use thereof**

(71) Applicant: Roche Diagnostics GmbH, 68305 Mannheim (DE)
(72) Inventor: Swart, Guido, Dr., 6525 JW Nijmegen (NL); Weidle, Ulrich H., Dr., 80336 München (DE)

(57) **Abstract**

The invention comprises an isolated nucleic acid molecule which hybridizes under stringent conditions to SEQ ID NO:1 and the complementary sequence. This nucleic acid molecule is a new gene with reduced expression in metastatic human melanoma cells.

SEQUENCE LISTING

(1) GENERAL INFORMATION:

(i) APPLICANT:
(A) NAME: BOEHRINGER MANNHEIM GMBH
(B) STREET: Sandhofer Str. 116
(C) CITY: Mannheim
(E) COUNTRY: Germany
(F) POSTAL CODE (ZIP): D-68305
(G) TELEPHONE: 08856/60-3446
(H) TELEFAX: 08856/60-3451

(ii) TITLE OF INVENTION: New gene with reduced expression in
metastatic human melanoma cells and the protein coded
thereby, methods of production and use thereof

(iii) NUMBER OF SEQUENCES: 4

(iv) COMPUTER READABLE FORM:
(A) MEDIUM TYPE: Floppy disk
(B) COMPUTER: IBM PC compatible
(C) OPERATING SYSTEM: PC-DOS/MS-DOS
(D) SOFTWARE: PatentIn Release #1.0, Version #1.30B (EPO)

(2) INFORMATION FOR SEQ ID NO: 1:

(i) SEQUENCE CHARACTERISTICS:
(A) LENGTH: 3456 base pairs
(B) TYPE: nucleic acid
(C) STRANDEDNESS: single
(D) TOPOLOGY: linear

(ii) MOLECULE TYPE: cDNA

(ix) FEATURE:
(A) NAME/KEY: CDS
(B) LOCATION:388..1848

(xi) SEQUENCE DESCRIPTION: SEQ ID NO: 1:

TTGAGAAGCT GGGTGAATGA ATGGTGGGAC CATTTCTGAC GTAGAAAACC CAGGAACAGG 60

AGTGGGCTCA TAGGGAAGGG GATAGTTTGC AAGGACTGTG TGATGGCCAA GTGGAGGCAT 120

CCAGTAGGCA GAGATGCGGG TCTGGGCCAG ACAGCAGATC AGGGGGCTTG AGGTTAAAGG 180

TGGTTTGGTG TGTGGGCACA GAAGACGTAA GAGAGAGGGC CTGGGACAGA ACCTGGGGAG 240

CGCCAGCAGC CAGGAAACAG GCAGAGGAGG TTGAGAGAAG GCACACTGCA GCCACTACAG 300

AAATGAGCTG GTTTGTCTAC AGATTTTGAG CGTTCGAAGT TGACCCCTGA CTAAGTATAC 360

TTTGCTGCTC CCTCAGCCTT TGAAAAA ATG TCT GTC ACA TAT GAT GAT TCC 411
Met Ser Val Thr Tyr Asp Asp Ser
1               5

GTT GGA GTA GAA GTG TCC AGC GAC AGC TTC TGG GAG GTC GGG AAC TAC 459
Val Gly Val Glu Val Ser Ser Asp Ser Phe Trp Glu Val Gly Asn Tyr
    10                  15                  20

AAG CGG ACT GTG AAG CGG ATC GAC GAT GGC CAC CGC CTG TGC AGC GAC 507
Lys Arg Thr Val Lys Arg Ile Asp Asp Gly His Arg Leu Cys Ser Asp
25                  30                  35                  40

CTC ATG AAC TGC CTG CAT GAG CGG GCG CGC ATC GAG AAG GCG TAT GCG 555
Leu Met Asn Cys Leu His Glu Arg Ala Arg Ile Glu Lys Ala Tyr Ala
                45                  50                  55

CAG CAG CTC ACT GAG TGG GCC CGG CGC TGG AGG CAG CTT GTG GAG AAA 603
Gln Gln Leu Thr Glu Trp Ala Arg Arg Trp Arg Gln Leu Val Glu Lys
            60                  65                  70

GGG CCC CAG TAC GGG ACC GTG GAG AAG GCC TGG ATG GCC TTC ATG TCC 651
Gly Pro Gln Tyr Gly Thr Val Glu Lys Ala Trp Met Ala Phe Met Ser
        75                  80                  85

GAG GCA GAG AGG GTG AGC GAG CTG CAC CTC GAG GTG AAG GCC TCA CTG 699
Glu Ala Glu Arg Val Ser Glu Leu His Leu Glu Val Lys Ala Ser Leu
    90                  95                  100

ATG AAC GAT GAC TTC GAG AAG ATC AAG AAC TGG CAG AAG GAA GCC TTT 747
Met Asn Asp Asp Phe Glu Lys Ile Lys Asn Trp Gln Lys Glu Ala Phe
105                 110                 115                 120

CAC AAG CAG ATG ATG GGC GGC TTC AAG GAG ACC AAG GAA GCT GAG GAC 795
His Lys Gln Met Met Gly Gly Phe Lys Glu Thr Lys Glu Ala Glu Asp
                125                 130                 135

GGC TTT CGG AAG GCA CAG AAG CCC TGG GCC AAG AAG CTG AAA GAG GTA 843
Gly Phe Arg Lys Ala Gln Lys Pro Trp Ala Lys Lys Leu Lys Glu Val
            140                 145                 150

GAA GCA GCA AAG AAA GCC CAC CAT GCA GCG TGC AAA GAG GAG AAG CTG 891
Glu Ala Ala Lys Lys Ala His His Ala Ala Cys Lys Glu Glu Lys Leu
        155                 160                 165

GCT ATC TCA CGA GAA GCC AAC AGC AAG GCA GAC CCA TCC CTC AAC CCT 939
Ala Ile Ser Arg Glu Ala Asn Ser Lys Ala Asp Pro Ser Leu Asn Pro
    170                 175                 180

GAA CAG CTC AAG AAA TTG CAA GAC AAA ATA GAA AAG TGC AAG CAA GAT 987
Glu Gln Leu Lys Lys Leu Gln Asp Lys Ile Glu Lys Cys Lys Gln Asp
185                 190                 195                 200

GTT CTT AAG ACC AAA GAG AAG TAT GAG AAG TCC CTG AAG GAA CTC GAC 1035
Val Leu Lys Thr Lys Glu Lys Tyr Glu Lys Ser Leu Lys Glu Leu Asp
                205                 210                 215

CAG GGC ACA CCC CAG TAC ATG GAG AAC ATG GAG CAG GTG TTT GAG CAG 1083
Gln Gly Thr Pro Gln Tyr Met Glu Asn Met Glu Gln Val Phe Glu Gln
            220                 225                 230

TGC CAG CAG TTC GAG GAG AAA CGC CTT CGC TTC TTC CGG GAG GTT CTG 1131
Cys Gln Gln Phe Glu Glu Lys Arg Leu Arg Phe Phe Arg Glu Val Leu
        235                 240                 245

CTG GAG GTT CAG AAG CAC CTA GAC CTG TCC AAT GTG GCT GGC TAC AAA 1179
Leu Glu Val Gln Lys His Leu Asp Leu Ser Asn Val Ala Gly Tyr Lys
    250                 255                 260

GCC ATT TAC CAT GAC CTG GAG CAG AGC ATC AGA GCA GCT GAT GCA GTG 1227
Ala Ile Tyr His Asp Leu Glu Gln Ser Ile Arg Ala Ala Asp Ala Val
265                 270                 275                 280

GAG GAC CTG AGG TGG TTC CGA GCC AAT CAC GGG CCA GGC ATG GCC ATG 1275
Glu Asp Leu Arg Trp Phe Arg Ala Asn His Gly Pro Gly Met Ala Met
                285                 290                 295

AAC TGG CCG CAG TTT GAG GAG TGG TCC GCA GAC CTG AAT CGA ACC CTC 1323
Asn Trp Pro Gln Phe Glu Glu Trp Ser Ala Asp Leu Asn Arg Thr Leu
            300                 305                 310

AGC CGG AGA GAG AAG AAG AAG GCC ACT GAC GGC GTC ACC CTG ACG GGC 1371
Ser Arg Arg Glu Lys Lys Lys Ala Thr Asp Gly Val Thr Leu Thr Gly
        315                 320                 325

ATC AAC CAG ACA GGC GAC CAG TCT CTG CCG AGT AAG CCC AGC AGC ACC 1419
Ile Asn Gln Thr Gly Asp Gln Ser Leu Pro Ser Lys Pro Ser Ser Thr
    330                 335                 340

CTT AAT GTC CCG AGC AAC CCC GCC CAG TCT GCG CAG TCA CAG TCC AGC 1467
Leu Asn Val Pro Ser Asn Pro Ala Gln Ser Ala Gln Ser Gln Ser Ser
345                 350                 355                 360

TAC AAC CCC TTC GAG GAT GAG GAC GAC ACG GGC AGC ACC GTC AGT GAG 1515
Tyr Asn Pro Phe Glu Asp Glu Asp Asp Thr Gly Ser Thr Val Ser Glu
                365                 370                 375

AAG GAC GAC ACT AAG GCC AAA AAT GTG AGC AGC TAC GAG AAG ACC CAG 1563
Lys Asp Asp Thr Lys Ala Lys Asn Val Ser Ser Tyr Glu Lys Thr Gln
            380                 385                 390

AGC TAT CCC ACC GAC TGG TCA GAC GAT GAG TCT AAC AAC CCC TTC TCC 1611
Ser Tyr Pro Thr Asp Trp Ser Asp Asp Glu Ser Asn Asn Pro Phe Ser
        395                 400                 405

TCC ACG GAT GCC AAT GGG GAC TCG AAT CCA TTC GAC GAC GAC GCC ACC 1659
Ser Thr Asp Ala Asn Gly Asp Ser Asn Pro Phe Asp Asp Asp Ala Thr
    410                 415                 420

TCG GGG ACG GAA GTG CGA GTC CGG GCC CTG TAT GAC TAT GAG GGG CAG 1707
Ser Gly Thr Glu Val Arg Val Arg Ala Leu Tyr Asp Tyr Glu Gly Gln
425                 430                 435                 440

GAG CAT GAT GAG CTG AGC TTC AAG GCT GGG GAT GAG CTG ACC AAG ATG 1755
Glu His Asp Glu Leu Ser Phe Lys Ala Gly Asp Glu Leu Thr Lys Met
                445                 450                 455

GAG GAC GAG GAT GAG CAG GGC TGG TGC AAG GGA CGC TTG GAC AAC GGG 1803
Glu Asp Glu Asp Glu Gln Gly Trp Cys Lys Gly Arg Leu Asp Asn Gly
            460                 465                 470

CAA GTT GGC CTA TAC CCG GCA AAT TAT GTG GAG GCG ATC CAG TGA 1848
Gln Val Gly Leu Tyr Pro Ala Asn Tyr Val Glu Ala Ile Gln *
        475                 480                 485

TGAGTCGGGG ACAGGCCAGC GGGGGGGACG GAGGCGGCGG GCCCAGGAGC CTCAGCCAGC 1908

CACGTGGGCA TCCACTCCTT TTCCTGCAAG AGATGATGGT TCCATTGCTC TTGGCTTCAT 1968

GGTGTTCCTG GAAGGCAGAT GAGCTGGTCA TTTCGCCTGG GACTCGGCAC CTTTCCGAGT 2028

GCAGCTGGAG GGATCTGAGC GCAGGAAGAC GCAGAACAAC AGAAATAGCC GCCCCTCCCC 2088

GCCCACTGTG CCTGTTGGCC TATCATAGAT CTCTATGTTC TTGACTTTGT CTCTCCTTTC 2148

CGAGTCAATG GTGGGTTACA CTGATCTTGT TCCACTGATT ACTCTCTCTG ACGAGTCCAT 2208

CACCTGCAAC TTAAATGAAC AAGCTTACAT CCCATTTTGA GTGAAGATTT TGAGGTTTTT 2268

AATTTAAAGG CTGTGTACAG TTATACTTTT TTATACACCT GTTCATTTCT ACTTAAATTA 2328

TGGCACAGAT TGATGCGCAC CAGTCTTGAG GAAACGATCT CCCTATTCCC TTACCCTGTT 2388

ACTCAGCCAC GCCGTGTGTA GGCTTAGCCT CAGGTGGCAG ATGTTTGAGA AAAGGAATTA 2448

TGCCAGGAAG GTGGGACCGG GTTATGGTCG GGTTTCTATT GGGAATGCTC TTTGTGCTTT 2508

TGGGCATCTG AATGGAAGCT TTACATAGAA CCTTAGGTAG AACTCCCCCA AATCGCCATA 2568

TTTAAAAATT ATTTTCACTC TATTCTTGCT TAAAACTGTA CTCTTTTGCA AATTAACAAT 2628

TTTATCACTG ATTCAGAGTT AAAAAGAAGA CTAACTTTTC AAGCAAATGC ATCTGTAAAG 2688

ATGCTTTAGA TTAGACTGTC ATGTCTCAGT GTCTATCTGT ATATATTATT TGATATTCAG 2748

AGAATCTAAA GCACTCGTCT ACTGTTTTAA TGAGATTTAA CAGCTTTTAA CAGTGAGTTT 2808

CGTTTGTAAA CTGCTTGAAG TCTGTGGCAT TCAGGCACAC ATCTGGCTGG CCGGCTGGGT 2868

CTCCTCCCGG GCTCAGTGGG CCTGGGGCCT CTCTGACGTG GTGCCTGCTG GAGGGAGGCT 2928

CGTCGTCACC AGCTGACTGC TGGTCCGGCT TCTGACCGGC CTTTGTCCTG GCTCCGTAGC 2988

AGAACACTGT AAAAGTGCCC GCGTCTTTGC AGTAGTTGCA GATTTCAGTC GTCGTGTTAC 3048

TTGTGCACAA ACAGAAGCTG GGTCTTACCC GCAGCACGAG TGTCTCGGGC TGCCCGGAGT 3108

CGCCCGGGAG CAGGTGCTGC AGCCAGAGTT ACGCGGGGGC CACGCGGGCC GGCGGGGGTG 3168

GGGGGAACGT GGGGGAACCT GTGTTTCACG TGACTCAGCA GTGCCCGCCG CCGTCACCAG 3228

CTATGCATTC ACTCCGTTTC CAGTGAGCAG ATGTCTTGCT TGGAAAGTGG ACCTGTGTCT 3288

GTGTCTGTCC TGAGAACTTA CCAGCAGAAA TCCTCATTTC TGTGCTACGG ATTTACCAAA 3348

AATTGTCAAG TCTTTTTCAG TTTAACAGTT CCTTTAAATG TGTAGTATTT GAGGAAAAAA 3408

ATCAATAAAC AGTTGATCTC GTGCATATGG AAAAAAAAAA AAAAAAAA 3456

(2) INFORMATION FOR SEQ ID NO: 2:

(i) SEQUENCE CHARACTERISTICS:
(A) LENGTH: 487 amino acids
(B) TYPE: amino acid
(D) TOPOLOGY: linear

(ii) MOLECULE TYPE: protein

(xi) SEQUENCE DESCRIPTION: SEQ ID NO: 2:

Met Ser Val Thr Tyr Asp Asp Ser Val Gly Val Glu Val Ser Ser Asp
1               5                   10                  15

Ser Phe Trp Glu Val Gly Asn Tyr Lys Arg Thr Val Lys Arg Ile Asp
            20                  25                  30

Asp Gly His Arg Leu Cys Ser Asp Leu Met Asn Cys Leu His Glu Arg
        35                  40                  45

Ala Arg Ile Glu Lys Ala Tyr Ala Gln Gln Leu Thr Glu Trp Ala Arg
    50                  55                  60

Arg Trp Arg Gln Leu Val Glu Lys Gly Pro Gln Tyr Gly Thr Val Glu
65                  70                  75                  80

Lys Ala Trp Met Ala Phe Met Ser Glu Ala Glu Arg Val Ser Glu Leu
                85                  90                  95

His Leu Glu Val Lys Ala Ser Leu Met Asn Asp Asp Phe Glu Lys Ile
            100                 105                 110

Lys Asn Trp Gln Lys Glu Ala Phe His Lys Gln Met Met Gly Gly Phe
        115                 120                 125

Lys Glu Thr Lys Glu Ala Glu Asp Gly Phe Arg Lys Ala Gln Lys Pro
    130                 135                 140

Trp Ala Lys Lys Leu Lys Glu Val Glu Ala Ala Lys Lys Ala His His
145                 150                 155                 160

Ala Ala Cys Lys Glu Glu Lys Leu Ala Ile Ser Arg Glu Ala Asn Ser
                165                 170                 175

Lys Ala Asp Pro Ser Leu Asn Pro Glu Gln Leu Lys Lys Leu Gln Asp
            180                 185                 190

Lys Ile Glu Lys Cys Lys Gln Asp Val Leu Lys Thr Lys Glu Lys Tyr
        195                 200                 205

Glu Lys Ser Leu Lys Glu Leu Asp Gln Gly Thr Pro Gln Tyr Met Glu
    210                 215                 220

Asn Met Glu Gln Val Phe Glu Gln Cys Gln Gln Phe Glu Glu Lys Arg
225                 230                 235                 240

Leu Arg Phe Phe Arg Glu Val Leu Leu Glu Val Gln Lys His Leu Asp
                245                 250                 255

Leu Ser Asn Val Ala Gly Tyr Lys Ala Ile Tyr His Asp Leu Glu Gln
            260                 265                 270

Ser Ile Arg Ala Ala Asp Ala Val Glu Asp Leu Arg Trp Phe Arg Ala
        275                 280                 285

Asn His Gly Pro Gly Met Ala Met Asn Trp Pro Gln Phe Glu Glu Trp
    290                 295                 300

Ser Ala Asp Leu Asn Arg Thr Leu Ser Arg Arg Glu Lys Lys Lys Ala
305                 310                 315                 320

Thr Asp Gly Val Thr Leu Thr Gly Ile Asn Gln Thr Gly Asp Gln Ser
                325                 330                 335

Leu Pro Ser Lys Pro Ser Ser Thr Leu Asn Val Pro Ser Asn Pro Ala
            340                 345                 350

Gln Ser Ala Gln Ser Gln Ser Ser Tyr Asn Pro Phe Glu Asp Glu Asp
        355                 360                 365

Asp Thr Gly Ser Thr Val Ser Glu Lys Asp Asp Thr Lys Ala Lys Asn
    370                 375                 380

Val Ser Ser Tyr Glu Lys Thr Gln Ser Tyr Pro Thr Asp Trp Ser Asp
385                 390                 395                 400

Asp Glu Ser Asn Asn Pro Phe Ser Ser Thr Asp Ala Asn Gly Asp Ser
                405                 410                 415

Asn Pro Phe Asp Asp Asp Ala Thr Ser Gly Thr Glu Val Arg Val Arg
            420                 425                 430

Ala Leu Tyr Asp Tyr Glu Gly Gln Glu His Asp Glu Leu Ser Phe Lys
        435                 440                 445

Ala Gly Asp Glu Leu Thr Lys Met Glu Asp Glu Asp Glu Gln Gly Trp
    450                 455                 460

Cys Lys Gly Arg Leu Asp Asn Gly Gln Val Gly Leu Tyr Pro Ala Asn
465                 470                 475                 480

Tyr Val Glu Ala Ile Gln *
                485

(2) INFORMATION FOR SEQ ID NO: 3:

(i) SEQUENCE CHARACTERISTICS:
(A) LENGTH: 19 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer T7F3"

(xi) SEQUENCE DESCRIPTION: SEQ ID NO: 3:

TGGAACCATC ATCTCTTGC 19

(2) INFORMATION FOR SEQ ID NO: 4:

(i) SEQUENCE CHARACTERISTICS:
(A) LENGTH: 18 base pairs
(B) TYPE: nucleic acid
(C) STRANDEDNESS: single
(D) TOPOLOGY: linear

(ii) MOLECULE TYPE: other nucleic acid
(A) DESCRIPTION: /desc = "primer JG18f"

(xi) SEQUENCE DESCRIPTION: SEQ ID NO: 4:

GAAGGACGAC ACTAAGGC 18

## Description

The present invention relates to the new gene rmm-1 (gene reduced in metastatic melanoma cells), the protein coded thereby, and their use for diagnostics and therapeutics, especially in the field of cancer. In particular, the invention relates to the diagnosis of the lack of gene grmm-1 in mammalian, especially in malignant melanoma cells, and gene therapy methods to restore grmm-1 and its functions in mammalian cells, especially in melanoma cells.

Genes with reduced expression in metastatic cells are potential tumor suppressor genes. Tumor suppressor genes are typically thought of as genes whose expression is reduced or lost in cancer cells (Knudson, Proc. Natl. Acad. Sci. USA 19 (1993) 10914-10921). The lack of expression results from mutations in the genes encoding their proteins and the related regulatory sequences. Since these proteins are believed to participate in suppression of cell growth and promotion of cell differentiation and thereby act as negative growth regulators, loss of their expression in tumor cells leads to the increased cell proliferation observed and contributes to malignant transformation. As negative growth regulators, tumor suppressor gene products are likely to have also normal functions critical to the development of differentiated tissues. In this respect, tumor suppressor genes may have an important role in the growth arrest necessary for the onset of cellular differentiation as growth regulation is a normal feature of development and differentiation. An overview of tumor suppressor genes is given by, e.g., Gutmann, D.H., Int. J. Dev. Biol. 39 (1995) 895-907.

Inactivation of tumor suppressor genes appears to be a predominant genetic event in the genesis and progression of many tumors. In normal cells, these genes are thought to be involved in the regulation of cell proliferation and differentiation (Fearon, E., and Vogelstein, B., Cell 61 (1990) 759-767). Inactivating mutations and deletions of tumor suppressor genes may therefore release normal growth constraints and may result in activation of the metastatic potential or progression of tumor cells.

Although malignant melanoma accounts for less than 5% of all cancers, it is among the most lethal because of its extremely high metastatic propensity (Balch, C.M., et al., Cutaneous Melanoma, Philadelphia: Lippincott, J.B., 1992). All melanocytic lesions derive from common melanocytes, which can degenerate through several nevus stages into a superficial primary melanoma in radial growth phase (PM-RGP). The vertical growth phase (PM-VGP) is the deeper, more invasive part of the primary tumor, which develops from PM-RGP melanoma and has metastatic potential (Herlyn, M., et al., Lab. Invest. 56 (1987) 416-474; Clark, W.H., et al., Hum. Pathol. 15 (1984) 1147-1165). Many molecular changes in tumor cells have been implicated as factors contributing to tumor metastasis. These changes, for example, include "gain-of function", such as autocrine expression of a growth factor or its receptor and expression of various cell adhesion molecules, as well as "loss-of-function", such as the inactivation of tumor suppressor genes, and loss of responsiveness to inhibitory growth factors (Kerbel, R.S., Adv. Cancer Res. 55 (1990) 87-132).

A number of molecular markers associated with subsequent stages of melanocytic tumors and derived melanoma cells have been described (for review, see Weterman, M.A.J., et al., Lab Invest 70 (1994) 593-608). In the last few years there was reported the isolation of several potential progression markers, e.g., calcyclin (Weterman, M.A.J., et al., Cancer Res. 52 (1992) 1291-1296; Weterman, M.A.J., et al., Cancer Res. 53 (1993) 6061-6066), thymosin-β10 (Weterman, M.A.J., et al., Int. J. Cancer 53 (1993) 278-284), nma (Degen, W.G.J., et al., Int. J. Cancer 65 (1996) 460-465), nmb (Weterman, M.A.J., et al., Int. J. Cancer 60 (1995) 73-81) and nmd (van Groningen, J.J.M., et al., Cancer Res. 55 (1995) 6237-6243; van Groningen, J.J.M., FEBS Lett. 404 (1997) 82-86). The availibility of a large panel of markers may allow a better discrimination between different stages of melanoma progression and a more refined diagnosis in the future.

It is an object of the invention to provide a novel tumor suppressor gene and its use. The invention therefore comprises an isolated nucleic acid molecule (grmm-1) with reduced expression in metastatic human melanoma cells and/or the expression of which is incompatible with metastasis of tumor cells, especially melanoma cells,
a) with the nucleic acid sequence SEQ ID NO:1, or
b) which hybridizes under stringent conditions with said nucleic acid or a nucleic acid complementary to (a), or
c) which hybridizes under stringent conditions with the nucleic acid molecule of a) or b) if there was no degeneracy of the genetic code,
   whereby hybridization in b) and c) is effected at least with the nucleic acid fragment of 1393 to 1539 of SEQ ID NO:1 or the complementary fragment.

The invention further comprises a recombinant polypeptide which
a) is coded by the DNA sequence shown in SEQ ID NO:1,
b) is coded by DNA sequences which hybridize under stringent conditions with said DNA or the complementary DNA, or,
c) is coded by DNA sequences which, if there was no degeneracy of the genetic code, would hybridize under stringent conditions with the nucleic acid fragments of (b),
   whereby hybridization is effected in b) and c) at least with the nucleic acid fragment of 1393 to 1539 of SEQ ID NO:1 or the complementary fragment.

The polypeptide can be defined by its DNA sequence and by the amino acid sequence derived therefrom. The isolated grmm-1 polypeptide can occur in natural allelic variations which differ from individual to individual. Such variations of the amino acids are usually amino acid substitutions. However, they may also be deletions, insertions or additions of amino acids to the total sequence. Preferably the sequence contains the fragment of nucleic acids 1393 to 1539 of SEQ ID NO:1. The grmm-1 protein according to the invention - depending, both in respect of the extent and type, on the cell and cell type in which it is expressed- can be in glycosylated or non-glycosylated form. Polypeptides with tumor-suppressive activity can easily be identified by a tumor progression inhibition assay using carcinoma cells expressing said polypeptides and measuring the proliferation capacity and apoptosis in relation to carcinoma cells not expressing said polypeptides.

"Polypeptide with grmm-1 activity or grmm-1" means also proteins with minor amino acid variations but with substantially the same grmm-1 activity. Substantially the same means that the activities are of the same biological properties and the polypeptides show preferably at least 75% homology (identity) in amino acid sequence. More preferably, the amino acid sequences are at least 90% identical.

The term "nucleic acid molecule" denotes a polynucleotide which can be, for example, a DNA, RNA, or derivatized active DNA or RNA. DNA and/or RNA molecules are preferred, however.

The term "hybridize under stringent conditions" means that two nucleic acid fragments are capable of hybridization to one another under standard hybridization conditions described in Sambrook et al., Molecular Cloning: A laboratory manual (1989) Cold Spring Harbor Laboratory Press, New York, USA.

More specifically, "stringent conditions" as used herein refer to hybridization in 6.0 x SSC at about 45°C, followed by a wash of 2.0 x SSC at 50°C. For selection of the stringency the salt concentration in the wash step can be selected, for example from about 2.0 x SSC at 50°C, for low stringency, to about 0.2 x SSC at 50°C, for high stringency. In addition, the temperature in the wash step can be increased from low stringency conditions at room temperatures, about 22°C, to high stringency conditions at about 65°C.

The term "isolated" as used throughout this application refers to a nucleic acid or polypeptide having a grmm-1 activity and is substantially free of cellular material or culture medium, when produced by recombinant DNA techniques, or chemical precursors or other chemicals, when synthesized chemically. An isolated nucleic acid is preferably free of sequences which naturally flank the nucleic acid (i.e. sequences located at the 5' and the 3' ends of the nucleic acid) in the organism from which the nucleic acid is derived.

grmm-1 can be purified after recombinant production by affinity chromatography using known protein purification techniques, including immunoprecipitation, gel filtration, ion exchange chromatography, chromatofocussing, isoelectric focussing, selective precipitation, electrophoresis, or the like.

The polypeptides according to the invention can also be produced by recombinant means, or synthetically. Non-glycosylated grmm-1 polypeptide is obtained when it is produced recombinantly in prokaryotes. With the aid of the nucleic acid sequences provided by the invention it is possible to search for the grmm-1 gene or its variants in genomes of any desired cells (e.g. apart from human cells, also in cells of other mammals), to identity these and to isolate the desired gene coding for the grmm-1 protein. Such processes and suitable hybridization conditions are known to a person skilled in the art and are described, for example, by Sambrook, J., et al., "Expression of cloned genes in E. coli" in Molecular Cloning: A laboratory manual (1989) Cold Spring Harbor Laboratory Press, New York, USA, and Hames, B.D., Higgins, S.G., Nucleic acid hybridisation - a practical approach (1985) IRL Press, Oxford, England. In this case the standard protocols described in these publications are usually used for the experiments.

The use of recombinant DNA technology enables the production of numerous active grmm-1 derivatives. Such derivatives can, for example, be modified in individual or several amino acids by substitution, deletion or addition. The derivatization can, for example, be carried out by means of site directed mutagenesis. Such variations can be easily carried out by a person skilled in the art (J. Sambrook, B.D. Hames, loc. cit.). It merely has to be ensured by means of the below-mentioned tumor cell growth inhibition assay that the characteristic properties of grmm-1 are preserved. The invention therefore in addition concerns a grmm-1 polypeptide which is a product of a prokaryotic or eukaryotic expression of an exogenous DNA.

With the aid of such nucleic acids coding for a grmm-1 protein, the protein according to the invention can be obtained in a reproducible manner and in large amounts. For expression in prokaryotic or eukaryotic organisms, such as prokaryotic host cells or eukaryotic host cells, the nucleic acid is integrated into suitable expression vectors, according to methods familiar to a person skilled in the art. Such an expression vector preferably contains a regulatable/inducible promoter. These recombinant vectors are then introduced for the expression into suitable host cells such as, e.g., E. coli as a prokaryotic host cell or Saccharomyces cerevisiae, Terato carcinoma cell line PA-1 sc 9117 (Büttner et al., Mol. Cell. Biol. 11 (1991) 3573-3583), insect cells, CHO or COS cells as eukaryotic host cells and the transformed or transduced host cells are cultured under conditions which allow an expression of the heterologous gene. The isolation of the protein can be carried out according to known methods from the host cell or from the culture supernatant of the host cell. Such methods are described for example by Ausubel I., Frederick M., Current Protocols in Mol. Biol. (1992), John Wiley and Sons, New York. Also in vitro reactivation of the protein may be necessary if it is not found in soluble form in the cell culture.

The invention further comprises recombinant expression vectors which are suitable for the expression of grmm-1, recombinant host cells transfected with such expression vectors, as well as a process for the recombinant production of a protein which is coded by a gene grmm-1.

The invention further comprises a method for detecting a nucleic acid molecule of gene grmm-1, comprising incubating a sample (e.g., body fluids such as blood, cell lysates) with the isolated nucleic acid molecule according to the invention and determining hybridization under stringent conditions of said isolated nucleic acid molecule to a target nucleic acid molecule as a determination of presence of a nucleic acid molecule which is the grmm-1 gene and therefore a method for the identification of the metastatic potential and/or progression of tumor cells.

On the basis of the nucleic acids provided by the invention it is possible to provide a test which can be used to detect nucleic acids with reduced expression in metastatic human melanoma cells. Such a test can be carried out by means of nucleic acid diagnostics. In this case the sample to be examined is contacted with a probe that is selected from the group comprising
a) the nucleic acids shown in SEQ ID NO:1 or a nucleic acid sequence which is complementary thereto,
b) nucleic acids which hybridize under stringent conditions with one of the nucleic acids from a), whereby hybridization is preferably effected at least with the nucleic acid fragment of 1393 to 1539 of SEQ ID NO:1 or the complementary fragment, wherein
   the nucleic acid probe is incubated with the nucleic acid of the sample and the hybridization is detected optionally by means of a further binding partner for the nucleic acid of the sample and/or the nucleic acid probe.

A hybridization between the probe and nucleic acids from the sample indicates the presence of the RNA of such proteins. Such methods are known to a person skilled in the art and are described, for example, in WO 89/06698, EP-A 0 200 362, USP 2915082, EP-A 0 063 879, EP-A 0 173 251, EP-A 0 128 018.

In a preferred embodiment of the invention the coding nucleic acid of the sample is amplified before the test, for example by means of the known PCR technique. Usually a derivatized (labeled) nucleic acid probe is used within the framework of nucleic acid diagnostics. This probe is contacted with a denatured DNA or RNA from the sample which is bound to a carrier and in this process the temperature, ionic strength, pH and other buffer conditions are selected - depending on the length of the nucleic acid probe and the resulting melting temperature of the expected hybrid - such that the labeled DNA or RNA can bind to homologous DNA or RNA (hybridization see also Wahl, G.M., et al., Proc. Natl. Acad. Sci. USA 76 (1979) 3683-3687). Suitable carriers are membranes or carrier materials based on nitrocellulose (e.g., Schleicher and Schüll, BA 85, Amersham Hybond, C.), strengthened or bound nitrocellulose in powder form or nylon membranes derivatized with various functional groups (e.g., nitro groups) (e.g., Schleicher and Schüll, Nytran; NEN, Gene Screen; Amersham Hybond M.; Pall Biodyne).

Hybridizing DNA or RNA is then detected by incubating the carrier with an antibody or antibody fragment after thorough washing and saturation to prevent unspecific binding. The antibody or the antibody fragment is directed towards the substance incorporated during derivatization into the nucleic acid probe. The antibody is in turn labeled. However, it is also possible to use a directly labeled DNA. After incubation with the antibodies it is washed again in order to only detect specifically bound antibody conjugates. The determination is then carried out according to known methods by means of the label on the antibody or the antibody fragment.

The detection of the expression can be carried out for example as:
in situ hybridization with fixed whole cells, with fixed tissue smears and isolated metaphase chromosomes,
colony hybridization (cells) and plaque hybridization (phages and viruses),
Southern hybridization (DNA detection),
Northern hybridization (RNA detection),
serum analysis (e.g., cell type analysis of cells in the serum by slot-blot analysis),
after amplification (e.g., PCR technique).

Therefore the invention also includes a method for the detection of the metastatic potential of a melanoma cell comprising
a) incubating a sample of body fluid of a patient suffering from melanoma cancer, of melanoma cells, or of a cell extract or a cell culture supernatant of said melanoma cells, whereby said sample contains nucleic acids with a nucleic acid probe which is selected from the group consisting of
   (i) the nucleic acid shown in SEQ ID NO:1 or a nucleic acid which is complementary thereto and
   (ii) nucleic acids which hybridize with one of the nucleic acids from (i) and
b) detecting the hybridization by means of a further binding partner of the nucleic acid of the sample and/or the nucleic acid probe.

Preferably the nucleic acid probe is incubated with the nucleic acid of the sample and the hybridization is detected optionally by means of a further binding partner for the nucleic acid of the sample and/or the nucleic acid probe.

It is also preferred that hybridization is effected at least with the nucleic acid fragment of 1393 to 1539 of SEQ ID NO:1 or the complementary fragment.

The nucleic acids according to the invention are hence valuable prognostic markers in the diagnosis of the metastatic and progression potential of tumor cells of a patient.

The invention further comprises a method for producing a protein whose expression is incompatible with tumor metastasis and/or which is capable of suppressing tumor progression, by expressing an exogenous DNA in prokaryotic or eukaryotic host cells and isolation of the desired protein, wherein the protein
a) is coded by the DNA sequence shown in SEQ ID NO:1,
b) is coded by DNA sequences which hybridize under stringent conditions with said DNA or the complementary DNA, or
c) is coded by DNA sequences which, if there was no degeneracy of the genetic code, would hybridize under stringent conditions with the sequences defined in (a) and (b),
   whereby hybridization is effected in b) and c) at least with the nucleic acid fragment of 1393 to 1539 of SEQ ID NO:1 or the complementary fragment.

The protein can be isolated from the cells or the culture supernatant and purified by chromatographic means, preferably by ion exchange chromatography, affinity chromatography and/or reverse phase HPLC.

The invention further comprises an isolated protein according to the invention which is encoded by a nucleic acid molecule having the nucleotide sequence set forth in SEQ ID NO:1.

The present invention relates to the cloning and characterization of the gene grmm-1, which is especially characterized as a tumor progression gene, and to an inhibitor gene for metastatic potential of melanoma cells. The functional reduction of the gene according to the invention (grmm-1) promotes loss of contact inhibition and anchorage dependence in tumor cells. Therefore the loss of grmm-1 gene correlates with a more aggressive behavior of the tumor cells and also the potential of the formation of metastasis.

According to the invention grmm-1 nucleic acids and their expression products can be used to inhibit tumor progression/metastasis, preferably of melanoma tumors, in vivo, preferably by somatic gene therapy.

The following examples, references and sequence listing are provided to aid the understanding of the present invention, the true scope of which is set forth in the appended claims. It is understood that modifications can be made in the procedures set forth without departing from the spirit of the invention.

### Description of the Figures

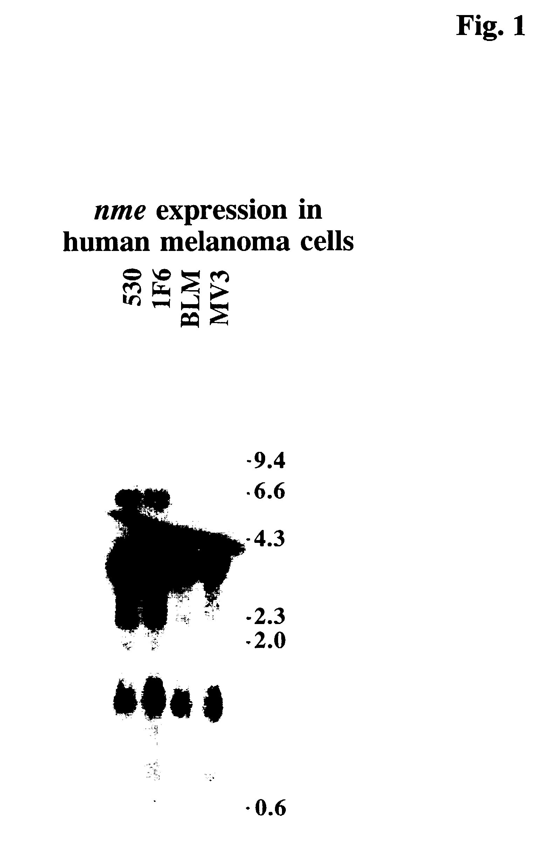

- **Figure 1**: Expression of grmm-1 (nme) in a panel of human melanoma cell lines by northern blot analysis. Total RNA samples (10 µg) from the indicated melanoma cell lines were loaded in each lane. The blot was hybridized with the grmm-1 cDNA insert. The molecular weight marker was lambda DNA digested with HindIII.

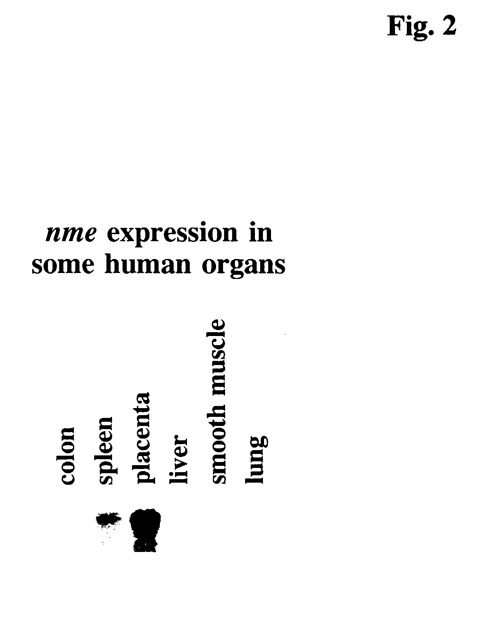

- **Figure 2**: Expression of grmm-1 (nme) in a selection of human organs by northern blot analysis. Total RNA samples (10 µg) from the indicated human organs were loaded in each lane. The blot was hybridized with the grmm-1 cDNA insert. The molecular weight marker was lambda DNA digested with HindIII.

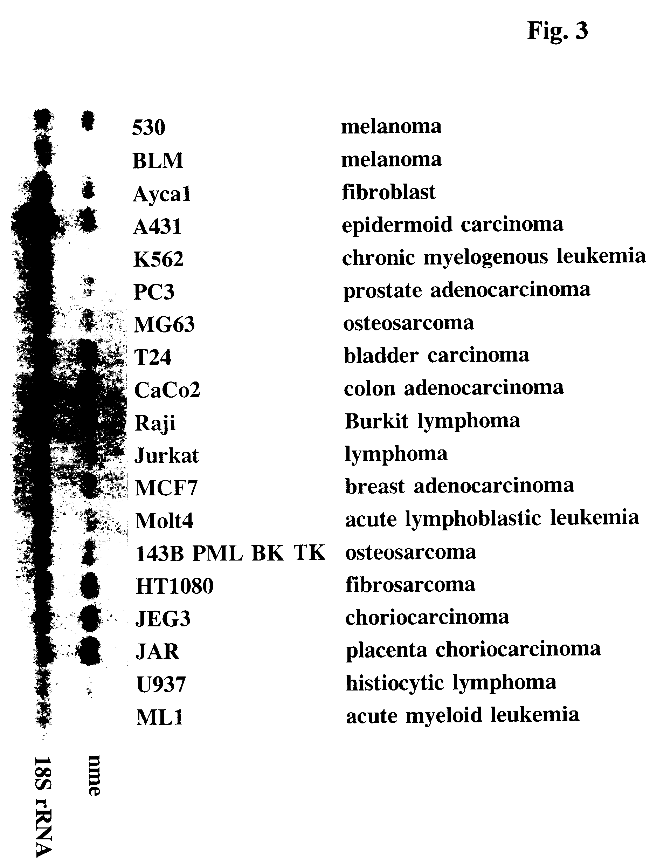

- **Figure 3**: Expression of grmm-1 (nine) in a collection of human (tumor) cell lines by northern blot analysis. Total RNA samples (10 µg) from the indicated human (tumor) cell lines were loaded in each lane. The blot was hybridized with the grmm-1 cDNA insert. The molecular weight marker was lambda DNA digested with HindIII. As a control for the amount of RNA loaded in each lane an 18S rRNA hybridization is shown.

### Biological materials:

Human melanoma cell lines MV1, MV3, BLM, 530 and 1F6 (van Muijen, G.N., et al., Clin. Exp. Metastasis 9 (1991) 259-272; van Muijen, G.N.P., et al., Int. J. Cancer 48 (1991) 85-91), T24 bladder carcinoma, PC-3 prostate carcinoma, HeLa cervix carcinoma MCF7 breast carcinoma, Caco-2 colon carcinoma, A-431 epidermoid carcinoma, HT-1080 fibrosarcoma, 143B PML BK TK osteosarcoma and MG-63 osteosarcoma were grown as monolayers, whereas U-937 histiocytic lymphoma, K-562 chronic myelogenous leukemia, KG-1 acute myelogenous leukemia, JEG-3 choriocarcinoma, JAR placenta choriocarcinoma, MOLT-4 acute lymphoblastic leukemia, Raji Burkitt lymphoma, and Jurkat lymphoma were grown in suspension. All cell lines were grown in Dulbecco's modified Eagle's medium (Gibco Laboratories) supplemented with 10% fetal calf serum (Gibco Laboratories), glutamine (2 mM), penicillin G (100 units per ml), streptomycin (100 µg/ml), and pyruvate (1 mM). Within the panel of melanoma cell lines, 1F6 and 530 represent non-metastasizing cell lines, with a metastasis frequency of less than 10% three months after subcutaneous inoculation into nude mice. The cell lines BLM and MV3 represent the highly metastatic phenotype, with over 50% metastasis frequency already after three weeks (van Muijen, G.N., et al., Clin. Exp. Metastasis 9 (1991) 259-272), Westphal et al., Br. J. Cancer 76 (1997) 561-570). Approximately 3x10⁶ cells were used for subcutaneous inoculation into nude mice (nu/nu BALB/c; Bomholtgaard, Ry, Denmark). Excision and processing of human tissues was performed as described before (Weterman, M.A.J., et al., Cancer Res. 53 (1993) 6061-6066).

### Example 1

### Isolation and identification of grmm-1

### RNA isolation and northern blot analysis:

Total RNA from human tissues and xenografts was isolated using the lithium-urea procedure (Auffray, C., and Rougeon, F., Eur. J. Biochem. 107 (1980) 303-314), whereas total RNA from cell lines was isolated using RNAzol solution (Tel-Test, Friendswood, TX). RNA oligodeoxythymidine selections were performed using oligo-deoxythymidine columns (type II, Coll. Research, Bedford, Massachusetts). Total RNA (10 µg) was glyoxylated (McMaster, G.K., and Carmichael, G.G., Proc. Natl. Acad. Sci. USA 74 (1977) 4835-4838), size fractionated on 1% agarose gels, and blotted onto Hybond N-plus (Amersham). To confirm that equal amounts were loaded in each lane, the blots were afterwards hybridized to a 18S rRNA probe.

### Construction of subtraction and cDNA libraries:

cDNA libraries were constructed from 5 µg of oligo-deoxythymidine selected RNA using a cDNA cloning kit (Invitrogen Corp.) or a lambda-Zap cDNA synthesis kit (Stratagene). Construction of the MV3-MV1 subtraction library with a subtractor kit (Invitrogen Corp.) was performed as described before (Weterman, M.A.J., et al., Int. J. Cancer 53 (1993) 278-284). The non metastasizing human melanoma cell line 530 was used for the construction of the 530 lambda-Zap cDNA library.

### DNA probes and hybridizations:

DNA probes were radiolabeled by the random prime labeling method (Feinberg, A.P., and Vogelstein, B., Anal. Biochem. 132 (1983) 6-13). Hybridization of the cDNA library was performed according to standard protocols (Sambrook et al., Molecular Cloning: A laboratory manual (1989) Cold Spring Harbor Laboratory Press, New York, USA).

### DNA sequencing and computer analysis:

The nucleotide sequence was determined by automated, fluorescent DNA sequencing. The complete cDNA sequence was compared with the GenEMBL database for homologies. DNA sequence analysis, including searches for motifs, alignments, and structure predictions, was performed using the CAMMSA programs MOTIFS, PILEUP, CLUSTAL V, BESTFIT, PEPTIDESTRUCTURE, PLOTSTRUCTURE, and MEMBRANE PROPENSITY which are all part of the Wisconsin Package V 7.0 (Devereux, J., et al., Nucleic Acids Res. 12 (1984) 387-395). Facilities for DNA sequence analysis were provided by the Dutch CAOS/CAMM centre.

### Expression of grmm-1 in human organs and tumor cell lines:

A Northern blot containing total RNA from gut, spleen, placenta, liver, smooth muscle, and lung, was hybridized with the grmm-1 cDNA insert as a probe (Figure 2). Expression of grmm-1 was detected in placenta, spleen and colon in decreasing order, and not detectable in liver, smooth muscle and lung. For further characterization a northern blot with total RNA from a number of human cell lines was screened with the grmm-1 cDNA insert (Figure 3). Within this collection expression levels comparable to non-metastatic melanoma cells (530) could be detected in human fibroblasts (Aycal), and in the tumor cell lines HT1080 (fibrosarcoma), JEG3 and JAR (choriocarcinoma), A431 (epidermoid carcinoma), T24 (bladder carcinoma, and CaCo2 (colon carcinoma). Most other cell lines had lower, but still detectable expression of grmm-1 transcripts. These data indicate that expression of grmm-1 is not restricted to melanocytic cells.

### DNA sequence analysis of grmm-1:

DNA sequence analysis of pJG384 revealed that the nine cDNA insert consists of 3460 nucleotides. The longest open reading frame (nucleotide positions 388-1746) encodes a 452 amino acids long polypeptide. Database searches for homology with known genes revealed similarities in the open reading frame with a murine gene (accession number X85124; 70% identity in 1160 bp overlap) and a chicken cDNA sequence (accession number Z50798; 77% identity in 1074 bp overlap; ref. Merilainen, J., et al., J. Biol. Chem. 272 (1997) 23278-23284). Additionally, the database search revealed 99% identity of the 3'-terminal half of the grmm-1 cDNA insert (nucleotide positions 1738-3444) with a fragment of human chromosome 22 (accession number Z82199).

Alignment of the predicted proteins from human, mouse and chicken showed that some 40 amino acids were inserted in the human protein between positions 336 and 384, while the human ORE is truncated by more than 30 amino acids at its C-terminus. This truncation is remarkable since it apparently disrupts an SH3 domain which is present in both murine (amino acids 387-438) and chicken (amino acids 393-444) gene products. Interactions between SH3 domains and proline-rich regions in other proteins mediate specific protein-protein complex formation. Closer inspection of nucleotide sequences alignment revealed that the truncation is caused by a four nucleotide insertion (frameshift) in the cDNA-insert of pJG384. For a more detailed analysis of the truncation and its frequency we performed RT-PCR reactions (see below).

### Example 2

### Functional test of the grmm-1 cDNA as a tumor suppressor gene:

The tumor suppressive activity of the grmm-1 cDNA can be tested by introduction of either the entire coding region or parts of the coding region of the nine cDNA into melanoma cells:

The entire coding region or in frame parts of the coding region are cloned into the tetracycline-inducible expression vector pUHD10-3 (Gossen, M., and Bujard, H., Proc. Natl. Acad. Sci. USA 89 (1992) 5547-5551). Together with a selection marker the different constructs are then electroporated into carcinoma cells containing the rtTa-gene on plasmid pUHD172-1neo (Gossen, M., et al., Science 268 (1995) 1766-1769). In selected cell clones expression of the grmm-1 cDNA is induced by treatment of the cells with tetracycline or doxycycline. In vitro, tumor suppressive activity of the nine cDNA constructs are determined by measuring the proliferative capacity and apoptosis of induced cells compared to non-induced cells. The generated carcinoma cells are further investigated in an *in vivo* mouse model. Cells are injected into immune-deficient mice. Expression of the grmm-1 cDNA constructs in the carcinoma cells is induced by tetracycline when small tumor nodules are apparent. Tumor suppression (tumor growth inhibition, tumor regression) is monitored in the induced and non-induced state.

### Example 3

### Detection of mRNA in melanoma cells

In order to detect whether proteins are expressed in melanoma cells which are coded by nucleic acids which hybridize with SEQ ID NO:1 and consequently whether mRNA is present, it is possible on the one hand to carry out the established methods of nucleic acid hybridization such as Northern hybridization, in-situ hybridization, dot or slot hybridization and diagnostic techniques derived therefrom (Sambrook et al., Molecular Cloning: A laboratory manual (1989) Cold Spring Harbor Laboratory Press, New York, USA; Hames, B.D., Higgins, S.G., Nucleic acid hybridisation - a practical approach (1985) IRL Press, Oxford, England; WO 89/06698; EP-A 0 200 362; USP 2915082; EP-A 0 063 879; EP-A 0 173 251; EP-A 0 128 018. On the other hand it is possible to use methods from the diverse repertoire of amplification techniques using specific primers (PRC Protocols - A Guide to Methods and Applications (1990), publ. M.A. Innis, D.H. Gelfand, J.J. Sninsky, T.J. White, Academic Press Inc.; PCR - A Practical Approach (1991), publ. M.J. McPherson, P. Quirke, G.R. Taylor, IRL Press. The RNA for this is isolated from melanoma cells by the method of Chomcszynski and Sacchi, Anal. Biochem. 162 (1987) 156-159. 20 µg total RNA was separated on a 1.2% agarose formaldehyde gel and transferred onto nylon membranes (Amersham, Braunschweig, DE) by standard methods (Sambrook et al., Molecular Cloning: A laboratory manual (1989) Cold Spring Harbor Laboratory Press, New York, USA. The DNA sequence SEQ ID NO:1 was radioactively labeled as probes (Feinberg, A.P., and Vogelstein, B., Anal. Biochem. 137 (1984) 266-267). The hybridization was carried out at 68°C in 5 x SSC, 5 x Denhardt, 7% SDS/0.5 M phosphate buffer pH 7.0, 10% dextran sulfate and 100 µg/ml salmon sperm DNA. Subsequently the membranes were washed twice for one hour each time in 1 x SSC at 68°C and then exposed to X-ray film.

### RT-PCR Analysis of grmm-1 transcripts in melanoma cell lines:

grmm-1 showed elevated mRNA expression (3.5 kb) in the non-metastatic human melanoma cell lines and derived xenografts as compared to the highly metastatic cell lines (Figure 1). The grmm-1 cDNA insert in the vector pJG384 consists of 3460 nucleotides and contains an open reading frame of 452 amino acids.

RT-PCR reactions were performed on total RNA isolated from the human melanoma cell line 530. The two oligo-deoxynucleotide primers used in this reaction (antisense T7F3: 5'-TGGAACCATCATCTCTTGC-3' (SEQ ID NO:3), and sense JG18f: 5'-GAAGGACGACACTAAGGC-3' (SEQ ID NO:4)) yielded a DNA-fragment of 0.44 kb spanning the presumed insertions. Fragments from three independent RT-PCR reactions were cloned in the vector pGEM-T Easy (Promega). Subsequent DNA-sequencing of 21 different clones revealed that none of these clones contained the earlier detected insertion of four nucleotides present in the cDNA insert of pJG384. The longest open reading frame (SEQ ID NO:1: nucleotide positions 388-1845) encodes a 486 amino acids long polypeptide. The usual human grmm-1 gene product contains an intact SH3-domain from amino acid position 431 to its C-terminus.

### List of References

Auffray, C., and Rougeon, F., Eur. J. Biochem. 107 (1980) 303-314
Ausubel I., Frederick M., Current Protocols in Mol. Biol. (1992), John Wiley and Sons, New York
Balch, C.M., et al., Cutaneous Melanoma, Philadelphia: Lippincott, J.B., 1992
Büttner et al., Mol. Cell. Biol. 11 (1991) 3573-3583
Chomcszynski and Sacchi, Anal. Biochem. 162 (1987) 156-159
Clark, W.H., et al., Hum. Pathol. 15 (1984) 1147-1165
Degen, W.G.J., et al., Int. J. Cancer 65 (1996) 460-465
Devereux, J., et al., Nucleic Acids Res. 12 (1984) 387-395
EP-A 0 063 879
EP-A 0 128 018
EP-A 0 173 251
EP-A 0 200 362
Fearon, E., and Vogelstein, B., Cell 61 (1990) 759-767
Feinberg, A.P., and Vogelstein, B., Anal. Biochem. 132 (1983) 6-13
Feinberg, A.P., and Vogeistein, B., Anal. Biochem. 137 (1984) 266-267
Gossen, M., and Bujard, H., Proc. Natl. Acad. Sci. USA 89 (1992) 5547-5551
Gossen, M., et al., Science 268 (1995) 1766-1769
Gutmann, D.H., Int. J. Dev. Biol. 39 (1995) 895-907
Hames, B.D., Higgins, S.G., Nucleic acid hybridisation - a practical approach (1985) IRL Press, Oxford, England
Herlyn, M., et al., Lab. Invest. 56 (1987) 416-474
Kerbel, R.S., Adv. Cancer Res. 55 (1990) 87-132
Knudson, Proc. Natl. Acad. Sci. USA 19 (1993) 10914-10921
McMaster, G.K., and Carmichael, G.G., Proc. Natl. Acad. Sci. USA 74 (1977) 4835-4838
Merilainen, J., et al., J. Biol. Chem. 272 (1997) 23278-23284
PCR - A Practical Approach (1991), publ. M.J. McPherson, P. Quirke, G.R. Taylor, IRL Press
PRC Protocols - A Guide to Methods and Applications (1990), publ. M.A. Innis, D.H. Gelfand, J.J. Sninsky, T.J. White, Academic Press Inc.
Sambrook et al., Molecular Cloning: A laboratory manual (1989) Cold Spring Harbor Laboratory Press, New York, USA
USP 2915082
van Groningen, J.J.M., et al., Cancer Res. 55 (1995) 6237-6243
van Groningen, J.J.M., FEBS Lett. 404 (1997) 82-86
van Muijen, G.N., et al., Clin. Exp. Metastasis 9 (1991) 259-272
van Muijen, G.N.P., et al., Int. J. Cancer 48 (1991) 85-91
Wahl, G.M., et al., Proc. Natl. Acad. Sci. USA 76 (1979) 3683-3687
Westphal et al., Br. J. Cancer 76 (1997) 561-570
Weterman, M.A.J., et al., Cancer Res. 52 (1992) 1291-1296
Weterman, M.A.J., et al., Cancer Res. 53 (1993) 6061-6066
Weterman, M.A.J., et al., Int. J. Cancer 53 (1993) 278-284
Weterman, M.A.J., et al., Int. J. Cancer 60 (1995) 73-81
Weterman, M.A.J., et al., Lab Invest 70 (1994) 593-608
WO 89/06698

## Claims

1. An isolated nucleic acid molecule (rmm-1) which is capable of suppressing tumor progression and/or metastasis
a) with the nucleic acid sequence SEQ ID NO:1, or
b) which hybridizes under stringent conditions with said nucleic acid or a nucleic acid complementary to (a), or
c) which hybridizes under stringent conditions with the nucleic acid molecules of a) or b) if there was no degeneracy of the genetic code,
whereby hybridization in b) or c) is effected at least with the nucleic acid fragment of 1393 to 1539 of SEQ ID NO:1 or the complementary fragment.

2. A recombinant expression vector which is suitable for the expression of a nucleic acid molecule as claimed in claim 1.

3. A recombinant polypeptide which suppresses tumor progression and/or metastasis and
a) is coded by the DNA sequence shown in SEQ ID NO:1,
b) is coded by DNA sequences which hybridize under stringent conditions with said DNA or the complementary DNA, or
c) is coded by DNA sequences which, if there was no degeneracy of the genetic code, would hybridize under stringent conditions with the nucleic acid fragments of (b),
whereby hybridization in b) or c) is effected at least with the nucleic acid fragment of 1393 to 1539 of SEQ ID NO:1 or the complementary fragment.

4. A method for the production of a protein which has tumor suppressive activity, by expressing an exogenous DNA in prokaryotic or eukaryotic host cells and isolation of the desired protein, wherein the protein
a) is coded by the DNA sequence shown in SEQ ID NO:1,
b) is coded by DNA sequences which hybridize under stringent conditions with said DNA or the complementary DNA, or
c) is coded by DNA sequences which, if there was no degeneracy of the genetic code, would hybridize under stringent conditions with the sequences defined in (a) and (b),
whereby hybridization in b) or c) is effected at least with the nucleic acid fragment of 1393 to 1539 of SEQ ID NO:1 or the complementary fragment.

5. A method for the detection of the metastatic potential of a melanoma cell comprising
a) incubating a sample of body fluid of a patient suffering from melanoma cancer, of melanoma cells, or of a cell extract or a cell culture supernatant of said melanoma cells, whereby said sample contains nucleic acids with a nucleic acid probe which is selected from the group consisting of
(i) the nucleic acid shown in SEQ ID NO:1 or a nucleic acid which is complementary thereto and
(ii) nucleic acids which hybridize with one of the nucleic acids from (i) and
b) detecting the hybridization by means of a further binding partner of the nucleic acid of the sample and/or the nucleic acid probe.

6. The method of claim 5, wherein hybridization is effected at least with the nucleic acid fragment of 1393 to 1539 of SEQ ID NO:1 or the complementary fragment.

7. The method of claim 5 or 6, wherein the nucleic acid to be detected is amplified before the detection.
